# EUROPEAN PATENT APPLICATION

(11) **EP 4 293 034 A1**
(43) Date of publication of application: **20.12.2023**
(21) Application number: 22382582.9
(22) Date of filing: 17.06.2022
(51) Int. Cl.: C07H 15/26, A61P 17/18, C12P 7/6454, C12P 19/02

(54) **A NOVEL ACYLATED DERIVATE OF PHLORETIN AND ITS USE AS ANTIOXIDANT**

(71) Applicant: Consejo Superior de Investigaciones Científicas (CSIC), 28006 Madrid (ES); Asociación Centro de Investigación Cooperativa en Biociencias - CIC bioGUNE, 48160 Derio, Bizkaia (Bizkaia) (ES); Ghent University, 9000 Ghent (BE)
(72) Inventor: PLOU GASCA, Francisco José, Madrid (ES); GONZÁLEZ ALFONSO, José Luis, Madrid (ES); BALLESTEROS OLMO, Antonio, Madrid (ES); ALONSO MERINO, Cristina, Madrid (ES); CODERCH, Luisa, Madrid (ES); POVEDA CABANES, Ana, Derio, Bizkaia (Bizkaia) (ES); JIMÉNEZ BARBERO, Jesús, Derio, Bizkaia (Bizkaia) (ES); DESMET, Tom, Ghent (BE); UBIPARIP, Zorica, Ghent (BE)
(74) Representative: Pons

(57) **Abstract**

The present invention relates to a method for producing acylated derivate of polyphenols comprising α-glucosylation of the phloretin with a sucrose phosphorylase mutant from *T. thermosaccharolyticum* and acylation with the lipase from *Thermomyces lanuginosus,* obtaining a novel acylated derivate of phloretin, particularly the phloretin 4'-O-(6-O-lauryl)-α-D-glucopyranoside. Further, the present invention relates the use of phloretin 4'-O-(6-O-lauryl)-α-D-glucopyranoside as antioxidant.

## Description

The invention belongs to the technical field of chemical compounds, particularly antioxidants. The present invention relates to a method for producing acylated derivate of polyphenols comprising α-glucosylation of the phloretin with a sucrose phosphorylase mutant from *T. thermosaccharolyticum* and acylation with the lipase from *Thermomyces lanuginosus,* obtaining a novel acylated derivative of phloretin, the phloretin 4'-O-(6-O-lauroyl)-α-D-glucopyranoside.

### BACKGROUND ART

Phloretin (2',4',6'-trihydroxy-3-(4-hydroxyphenyl)-propiophenone) is a polyphenol belonging to the subgroup of dihydrochalcones. This compound is mainly present in most parts of the apple tree, including leaves, skin and pomace of apples. Previous reports stand out the notable phloretin antioxidant and biological activity. It is effective for antidiabetic applications through the inhibition of the glucose active transport (Shen, X., et al, 2020, Food & Function, 11 (1), 392-403*).*

Furthermore, phloretin could be useful for anticancer applications in combination with other current treatments, due to its ability to supress cancer cell migration (Hsiao, Y.-H., etal, 2019, Phytomedicine, 62, 152964*).* The antiviral activity against HIV-1 of this phenolic compound was related to its capacity to inhibit Gp41 transmembrane HIV-1 protein (Yin, S., et al, 2018, FEBS Letters, 592 (13), 2361-2377).

However, the bioavailability of phloretin is low because of its poor absorption. The ability of these compounds to pass through the skin barriers depends, in part, on their lipophilicity. The binding of a fatty acid to the molecule could enhance its affinity to the lipid membranes of the skin cells and, therefore, improve the absorption (Alonso, C., et al, 2015, Journal of Pharmacy and Pharmacology, 67 (7), 900-908*).*

The acylation of antioxidants with an alkyl chain can also improve some biological properties or even act as prodrug. In this context, resveratrol acylated analogues enhance the cell-grown inhibition of cancer prostate cells (Cardile, V., et al, 2005, Bioorganic Chemistry, 33 (1), 22-33)*.* It is described the direct enzymatic acylation of some phenolic aglycones.

However, there are not many articles about acylations on phenolic alcohols, because normally this occurs in a primary alcohol which some polyphenol possesses, such as hydroxytyrosol. In other cases, the authors described the use of phenolic acids as acyl donors for fatty alcohols. Some examples of phenolic alcohols acylation are reported by Torres P., et al, 2010, Journal of Agricultural and Food Chemistry, 58 (2), 807-813 with resveratrol and vitamin E, employing lipases from *Alcaligenes sp.* and *Candida antarctica.*

Other authors obtained a quercetin oleate ester with enhanced lipophilicity, using the lipase from *Candida antarctica* (Saik, A. Y., et al, 2017, Biotechnology Letters, 39 (2), 297-304*).* However, the acylation of phenolic aglycones is limited by the substrate affinity, the reaction times and/or the reaction yields.

Previously, the phloretin glycosylation and the skin absorption of the glucosides obtained (monoglucoside and diglucoside) have been described (Gonzalez-Alfonso, J. L., et al, 2021, Advanced Synthesis & Catalysis, 363 (n/a), 3079-3089*).* Phloretin and its monoglucoside displayed the same absorption pattern, without significant differences. On the other hand, the diglucoside derivative absorption was diminished, providing an interesting antioxidant and soluble compound to protect the external skin layers. However, if the objective is to increase the percutaneous absorption allowing the compound to penetrate through the skin barriers, the simple glycosylation is not normally a good strategy.

The acylation of plant polyphenols usually gives rise to low yields due to the limited reactivity of phenolic OHs. Further, the bioavailability of phloretin is low because of its poor absorption, which constraints its application in cosmetical and functional food preparations.

Nevertheless, the increase of the percutaneous absorption of phloretin (and other polyphenols) for cosmetic applications is still a challenge.

### DESCRIPTION OF THE INVENTION

The present invention refers a useful two-step strategy for the synthesis of acylated derivatives of polyphenols. The first step is the α-glucosylation by a sucrose phosphorylase mutant, which gives rise to an excellent yield and regioselectivity with many polyphenols, including phloretin, a bioactive dihydrochalcone mainly present in apples. The glucose moiety provides an acylation point for regioselective lipases, such as that from *Thermomyces lanuginosus.*

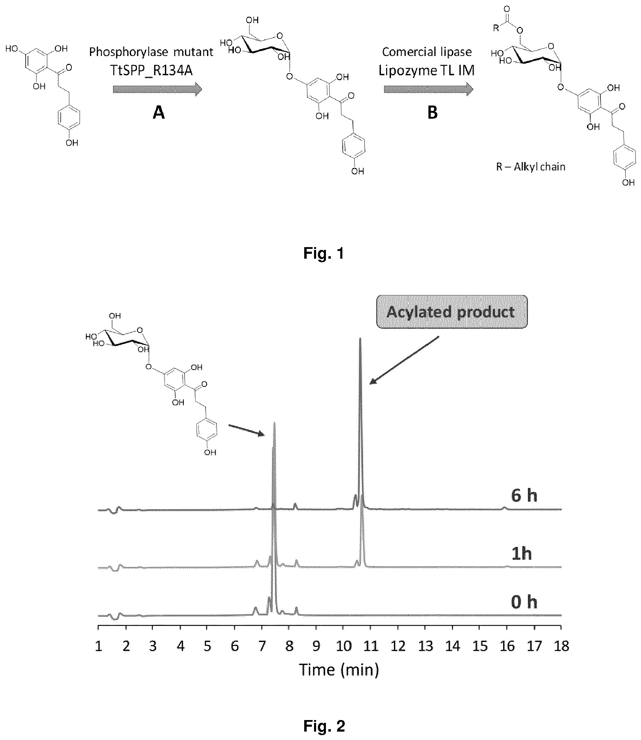

Using this strategy, a very high yield of acylated α-glucoside of phloretin has been obtained. First, the inventors have obtained the α-glucosylated derivative at 4' OH, the phloretin 4'-O-α-D-glucopyranoside, by α-glucosylation of the phloretin with a sucrose phosphorylase mutant from *T. thermosaccharolyticum* (TtSPP_R134A). Then, a regio-selective acylation of the phloretin 4'-O-α-D-glucopyranoside was performed with the lipase from *Thermomyces lanoginosus* (Lipozyme TL IM). The high regioselectivity of this enzyme to acylate in 6-OH glucose allowed to predict the molecular structure of the obtained phenolic ester. The reaction pathway is shown in Figure 1. The acylated derivative of phloretin 4'-O-α-D-glucopyranoside obtained, for example the phloretin 4'-O-(6-O-lauroyl)-α-D-glucopyranoside, is more efficient to penetrate through a skin model, which opens the door to develop tailor-made products for cosmetic applications.

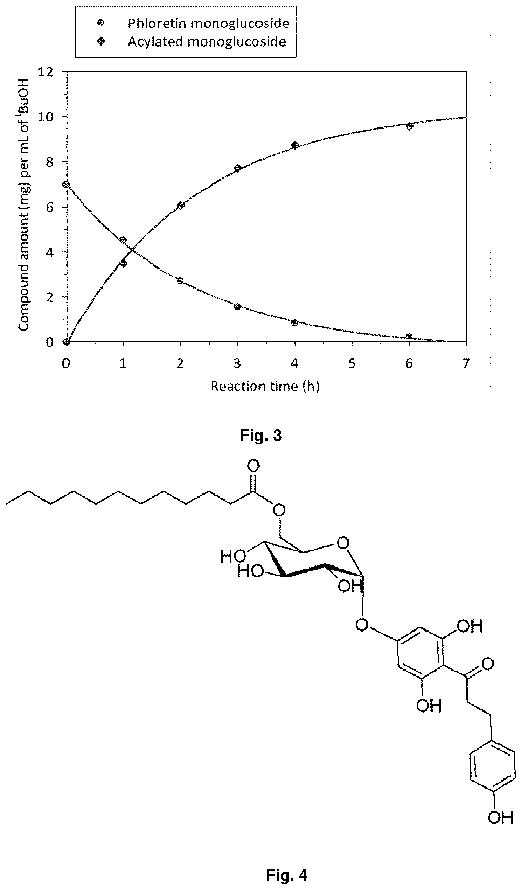

By MS and 2D-NMR it was confirmed that the acylation took place with high regioselectivity at the 6-OH of glucose, yielding the novel compound phloretin 4'-O-α-D-(glucopyranoside 6-laurate) (Figure 4). The antioxidant activity of the acyl-monoglucoside was evaluated measuring the Trolox Equivalent Antioxidant Capacity (TEAC) value on ABTS and DPPH (Table 2).

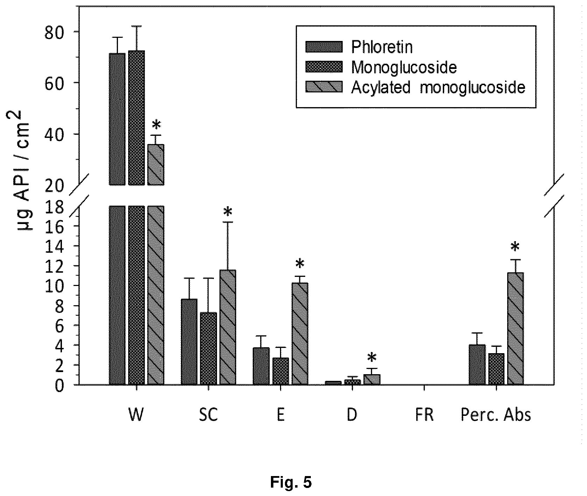

It is worth highlighting that the percutaneous absorption of this derivative -using a pig skin model-was 3-fold higher than the corresponding to phloretin and its α-glucoside (Figure 5). These facts make the acylglucoside of phloretin very promising for cosmeceutical applications and opens a strategy for the synthesis of other acylated flavonoids.

Thus, a first aspect of the invention is a novel compound, the acylated derivative of phloretin 4'-O-α-D-glucopyranoside, hereinafter "the compound of the invention", of formula (I): 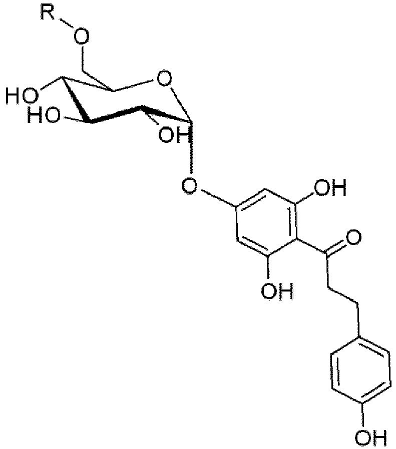 wherein R is a -(CO)-C₇-C₁₇ linear alkyl or a -(CO)-C₇-C₁₇ linear alkenyl group.

As used herein, the term "C₇-C₁₇ linear alkyl" refers to an aliphatic, linear hydrocarbon chain containing between 7 and 17 carbon atoms, such as, for example, linear heptyl, octyl, nonyl, decyl, undecyl, dodecyl, tridecyl, tetradecyl, pentadecyl, hexadecyl and heptadecyl.

In a preferred embodiment of the compound of the present invention, R is a -(CO)-C₇-C₁₇ linear alkyl group.

The term "C₇-C₁₇ linear alkenyl" refers to an aliphatic, linear hydrocarbon chain having at least one double bond and containing between 7 and 17 carbon atoms, such as, for example, linear heptenyl, octenyl, nonenyl, decenyl, undecenyl, dodecenyl, tridecenyl, tetradecenyl, pentadecenyl, hexadecenyl and heptadecenyl.

In a preferred embodiment of the compound of the present invention, R is an acyl group from a fatty acid comprising between 8 and 18 carbon atoms.

In a preferred embodiment of the compound of the present invention, R is a -(CO)-Cn linear alkyl group, wherein n is an integer selected from: 7, 9, 11, 13, 15 and 17.

"Cn linear alkyl" refers to an aliphatic, linear hydrocarbon chain containing n carbon atoms.

In a preferred embodiment of the compound of the present invention, R is a -(CO)-Cn linear alkenyl group, wherein n is 13, 15 or 17.

"Cn linear alkenyl" refers to an aliphatic, linear hydrocarbon chain having at least one double bond and containing n carbon atoms.

In a more preferred embodiment, the compound of the invention is an acylated derivative of phloretin 4'-O-α-D-glucopyranoside wherein R is a -(CO)-undecyl of formula (II), the phloretin 4'-O-(6-O-lauroyl)-α-D-glucopyranoside:

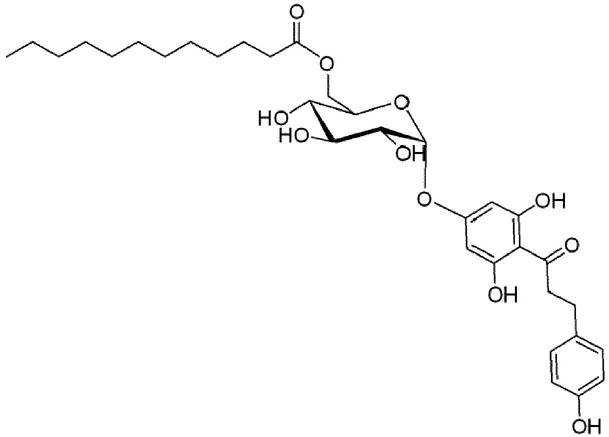

Another aspect of the present invention related to a composition comprising the compound of formula (I) or compound of formula (II) of the present invention, hereinafter "the composition of the invention".

In a preferred embodiment of the present invention, the composition is a cosmetic composition, comprising a cosmetically effective amount of the compound of the invention, and optionally, comprising a cosmetically acceptable excipient or vehicle.

As used in the present invention, the expression "cosmetic composition" refers to a formulation that has been adapted to administer a predetermined dose of one or more useful cosmetic agents to a cell, a group of cells, an organ, a tissue or an animal, particularly as antioxidant.

The term "cosmetically effective amount", as used herein, is understood to mean an amount capable of providing a cosmetic effect, and which can be determined by the person skilled in the art by commonly used means. The amount of the phloretin 4'-O-(6-O-lauroyl)-α-D-glucopyranoside that may be included in the cosmetic compositions according to the invention will vary depending on the subject and the particular mode of administration, at a concentration in the range between 1% and 10%v/v, preferably between 1 and 5% v/v, more preferably in 2% v/v.

The compositions of the invention also contain one or more additional acceptable excipients. By "acceptable excipient" is meant a inactive substance which is used to incorporate the active ingredient and which is acceptable from a toxicological point of view and to the manufacturing chemist from a physical/chemical point of view with respect to composition, formulation, stability, acceptance and bioavailability. The excipient or vehicle also includes any substance that serves to enhance the delivery and efficacy of the active ingredient within the composition. Examples of acceptable vehicles include one or more from the list comprising: water, saline, phosphate-buffered saline, dextrose, glycerol, ethanol and the like, as well as combinations thereof. In many cases, it will be preferable to include isotonic agents, for example, sugars, polyols such as mannitol, sorbitol or sodium chloride in the composition. Acceptable vehicles may further comprise minor amounts of auxiliary substances, such as wetting or emulsifying agents, preservatives or buffers, which improve the shelf life or efficacy of the compositions. Examples of suitable vehicles are well known in the literature. Examples of vehicles without limitation are a series of saccharides such as lactose, dextrose, sucrose, sorbitol, mannitol, xylitol, erythritol and maltitol; a series of starches such as corn starch, wheat starch, rice starch and potato starch; a series of cellulose such as cellulose, methyl cellulose, sodium carboxymethyl cellulose and hydroxypropyl methyl cellulose; and a series of fillers such as gelatin and polyvinylpyrrolidone. In some cases, a disintegrant such as cross-linked polyvinylpyrrolidone, agar, alginic acid or sodium alginate may be added.

The compositions optionally including one or more suitable diluents, fillers, salts, disintegrants, binders, lubricants, glidants, wetting agents, controlled release matrices, colorants/flavouring, carriers, excipients, buffers, stabilizers, solubilizers, and combinations thereof.

The cosmetic composition may further comprise adjuvants, excipients and other additives common in the cosmetic and dermatological field, such as but not limited to stabilizers, preservatives, antioxidants, solvents, perfumes, chelating agents, odor absorbers, chemical or mineral filters, mineral pigments, organic solvents, silicones, thickeners, softeners, emulsifiers, antifoaming agents, moisturizers, fragrances, surfactants, anionic, cationic, nonionic or amphoteric polymers or mixtures thereof, sequestering agents, propellants, acidifying or basifying agents, dyes, colorants, pigments or nanopigments, or any other ingredients normally used in cosmetic products and compositions.

In other preferred embodiment the cosmetic composition further comprises a second active ingredient, preferably wherein the second active ingredient is selected from the list consisting of: cinnamic acid, caffeic acid, gallic acid, resveratrol, pterostilbene, epigallocatequin gallate, quercetin, hesperetin, luteolin, baicalin, mangiferin, retinol, ascorbic acid, astaxanthin, tocopherol, and their derivatives.

The cosmetic composition of the present invention can be administered by any route that enables delivery of the compound of the invention to the site of action, such as oral routes, intraduodenal routes, parenteral injection (including intravenous, intraarterial, subcutaneous, intramuscular, intravascular, intraperitoneal or infusion), topical administration (e.g. transdermal application), rectal administration, via local delivery by catheter or stent or through inhalation. The compound can also be administered intraadiposally or intrathecally.

The compositions can be administered in solid, semi-solid, liquid or gaseous form, or may be in dried powder, such as lyophilized form.

In other preferred embodiment the composition of the invention is formulated for administration in liquid or solid form.

The cosmetic composition can be packaged in forms convenient for delivery, including, for example, solid dosage forms such as capsules, sachets, cachets, gelatines, papers, tablets, capsules, suppositories, pellets, pills, troches, and lozenges. The type of packaging will generally depend on the desired route of administration. Implantable sustained release formulations are also contemplated, as are transdermal formulations.

Aging skin is the result of more than just chronological age. Skin is exposed to environmental elements that cause radicals to form in the skin. These radicals attack the collagen layer of the skin and break it down, causing lines and wrinkles to appear. This process is commonly called photo-aging. Application of antioxidants can help prevent radical-induced damage in skin.

The present invention is also directed to a cosmetic or non-therapeutic process for treating radical-induced damage to skin by contacting the skin.

Other aspect of the present invention is the cosmetic use (non-therapeutic use) of the compound of the invention or cosmetic composition of the invention as an antioxidant, as described above.

As described above the compound of the invention, the phloretin 4'-O-(6-O-lauroyl)-α-D-glucopyranoside, is obtained by α-glucosylation of the phloretin with a sucrose phosphorylase mutant from *T. thermosaccharolyticum* and acylation with the lipase from *Thermomyces lanoginosus.*

So, another aspect of the invention relates to a method for producing acylated derivative of phloretin 4'-O-α-D-glucopyranoside (the compound of invention), hereinafter "the method of the invention", comprising:
(i) contacting the phloretin (2',4',6'-trihydroxy-3-(4-hydroxyphenyl)-propiophenone) with a mutant of the sucrose 6'-phosphate phosphorylase from *Thermoanaerobacterium thermosaccharolyticum,* in aqueous media and in presence of sucrose, wherein the said mutant of the sucrose 6'-phosphate phosphorylase comprises the amino acid sequence of SEQ ID NO: 2, and
(ii) contacting the 4'-O-α-D-glucopyranoside obtained in step (i) with an immobilized lipase from *Thermomyces lanuginosus,* in organic solvent and in presence of a fatty acid with a chain comprising between 8 to 18 carbon atoms or a derivative thereof, obtaining the acylated derivative of phloretin 4'-O-α-D-glucopyranoside.

The "phloretin", "2',4',6'-trihydroxy-3-(4-hydroxyphenyl)-propiophenone" or "3-(4-Hydroxyphenyl)-1-(2,4,6-trihydroxyphenyl)propan-1-one" is a member of the class of dihydrochalcones, a type of phenol, that is dihydrochalcone substituted by hydroxy groups at positions 4, 2', 4' and 6'. It has a role as a plant metabolite and an antineoplastic agent. Phloretin is a dihydrochalcone mainly present in most parts of apple trees, including the skin and pomace of apples, which possesses a notable antioxidant activity and stands out for a variety of biological properties. Thus, it displays an inhibitory effect on the active transport of glucose, which makes it an interesting compound as antidiabetic found in apples and other fruits.

The phloretin has the following chemical structure:

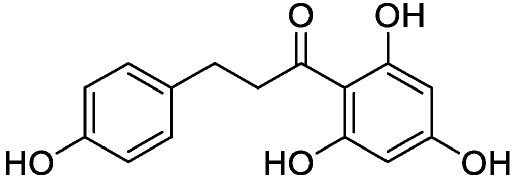

The method of the invention comprises the α-glucosylation of the phloretin with a sucrose phosphorylase from *Thermoanaerobacterium thermosaccharolyticum,* particularly the R134A mutant of the sucrose 6'-phosphate phosphorylase from *T*. *thermosaccharolyticum* (TtSPP_R134A).

The "sucrose 6'-phosphate phosphorylase" with activity according to EC:2.4.1.329, which catalyzes the reversible phosphorolysis of sucrose 6'-phosphate into alpha-D-glucose 1-phosphate (Glc1P) and D-fructose 6-phosphate, yielding the 4'-O-α-D-glucopyranoside. Particularly, this enzyme is isolated from the thermophilic bacterium *Thermoanaerobacterium thermosaccharoliticum.*

The sucrose 6'-phosphate phosphorylase from *Thermoanaerobacterium thermosaccharoliticum* (UniProt ID D9TT09, EC:2.4.1.329), comprises the amino acid sequence of SEQ ID NO: 1
SEQ ID NO: 1

MALKNKVQLITYPDSLGGNLKTLNDVLEKYFSDVFGGVHILPPFPSSGDRGFAPITV
SEIEPKFGTWYDIKKMAENFDILLDLMVNHVSRRSIYFQDFLKKGRKSEYADMFITL
DKLWKDGKPVKGDIEKMFLRRTLPYSTFKIEETGEEEKVWTIFGKTDPSEQIDLDVN
SHLVREFLLEVFKTFSNFGVKIVRLDAVGYVIKKIGTSCFFVEPEIVEFLDWAKGQA
ASYGIELLLEVHSQFEVQYKLAERGFLIYDFILPFTVLYTLINKSNEMLYHYLKNRP
INQFTMLDCHDGIPVKPDLDGLIDTKKAKEVVDICVQRGANLSLIVGDKVKSEDGFD
VHQINCTYVSALNCDDDAYLAARAIQFFTPGIPQVYYVGLLAGVNDFEAVKKTKEGR
EINRHNYGLKEIEESVQKNVVQRLLKLIRFRNEYEAFNGEFFIEDCRKDEIRLTWKK
DDKRCSLFIDLKTYKTIIDYINENGEEVKYLV

Particularly, the sucrose 6'-phosphate phosphorylase from *Thermoanaerobacterium thermosaccharoliticum* used in the method of the invention comprises a mutation wherein Arginine (R) is replaced with Alanine (A) at position 134 of the native enzymatic sequence (SEQ ID NO:1). The R134A mutant of sucrose 6'-phophate phosphorylase from *T. thermosaccharoliticum* (TtSPP_R134A) exhibits higher affinity for polyphenols than the native enzyme, caused by the increased size of the catalytic pocket.

Thus, the mutant of the sucrose 6'-phosphate phosphorylase from *T. thermosaccharolyticum* (TtSPP_R134A), comprises the amino acid sequence of SEQ ID NO: 2 (mutation A134 is indicated in bold & underlined).
SEQ ID NO: 2

MALKNKVQLITYPDSLGGNLKTLNDVLEKYFSDVFGGVHILPPFPSSGDRGFAPITV
SEIEPKFGTWYDIKKMAENFDILLDLMVNHVSRRSIYFQDFLKKGRKSEYADMFITL
DKLWKDGKPVKGDIEKMFLARTLPYSTFKIEETGEEEKVWTIFGKTDPSEQIDLDVN

SHLVREFLLEVFKTFSNFGVKIVRLDAVGYVIKKIGTSCFFVEPEIVEFLDWAKGQA
ASYGIELLLEVHSQFEVQYKLAERGFLIYDFILPFTVLYTLINKSNEMLYHYLKNRP
INQFTMLDCHDGIPVKPDLDGLIDTKKAKEVVDICVQRGANLSLIVGDKVKSEDGFD
VHQINCTYVSALNCDDDAYLAARAIQFFTPGIPQVYYVGLLAGVNDFEAVKKTKEGR
EINRHNYGLKEIEESVQKNVVQRLLKLIRFRNEYEAFNGEFFIEDCRKDEIRLTWKK
DDKRCSLFIDLKTYKTIIDYINENGEEVKYLV

In other preferred embodiment of the present invention the enzyme concentration ranges between 1 to 8 mg/mL, preferably between 2 to 6 mg/mL, more preferably the enzyme concentration is 4 mg/mL, which corresponded to a final activity of 5 U/mL measured by 2,2-Biquinoline-4,4-dicarboxylic acid (BCA) method (Gonzalez-Alfonso, J., et al, 2021, Advanced Synthesis & Catalysis, 363, 3079-30).

The α-glucosylation of the phloretin is carried out in aqueous media. The term "aqueous media" as used herein, refers to a medium, solution, dilution whose solvent is water, that is, the compounds of medium are dissolved in water. It is practice of routine for the skilled person in the art the suitable aqueous media for α-glucosylation. Examples of aqueous media include, with limitation to, MOPS buffer, phosphate buffer, acetate buffer or citrate buffer.

In a preferred embodiment of the method of the invention, the aqueous media is selected from the list consisting of: MOPS (3-(N-morpholino)propanesulfonic acid) buffer, phosphate buffer, acetate buffer and citrate buffer.

Particularly, the α-glucosylation of the phloretin is carried out in aqueous media and in presence of sucrose at a concentration between 50 and 500 g/L, preferably between 100 to 400, more preferably the sucrose concentration is 342 g/L (1 M).

Subsequently, the 4'-O-α-D-glucopyranoside obtained in the α-glucosylation of the phloretin with the TtSPP_R134A is acylated with an immobilized lipase from *Thermomyces lanuginosus,* in an organic solvent and in presence of a fatty acid with a chain comprising between 8 to 18 carbon atoms, obtaining the acylated derivative of phloretin 4'-O-α-D-glucopyranoside, the compound of the invention.

In the present invention, the "acylation" is the process of adding an acyl group to a compound, particularly to the 4'-O-α-D-glucopyranoside, catalyzed by a lipase from *Thermomyces lanuginosus.*

The "lipase" with activity according to EC 3.1.1.3 which hydrolyzes triglycerides into diglycerides and subsequently into monoglycerides and free fatty acids. The lipase of the method of the invention is from *Thermomyces lanuginosus.* The final activity was 1.75 IUN (Interestification Unit Novo) per millilitre of reaction medium. One Interesterification Unit Novo is defined as 0.01 w/w % converted tristearin/minute (initial rate) at specific conditions fixed by the manufacturer (Novozymes).

The acylation of the 4'-O-α-D-glucopyranoside in step (ii) is carried out in organic media or organic solvent. The term "organic media" or "organic solvent" as used herein, refers to a medium, solution, dilution which solvent is an organic solvent, that is, the compounds of medium are dissolved in organic medium. It is practice of routine for the skilled person in the art the suitable solvents for acylation. Examples of solvents include, with limitation to, *tert*-butyl alcohol, 2-methyl-2-butanol (*tert*-amyl alcohol), acetonitrile, ethanol, methanol or acetone.

In a preferred embodiment of the method of the invention, the organic solvent is selected from the list consisting of: *tert*-butyl alcohol solution, 2-methyl-2-butanol (*tert-*amyl alcohol), acetonitrile, ethanol, methanol and acetone, preferably *tert*-butyl alcohol.

The 4'-O-α-D-glucopyranoside is acylated with an immobilized lipase from *T*. *lanuginosus* in presence of a fatty acid, or derivative thereof, with a chain comprising between 8 to 18 carbon atoms.

The "fatty acid" is a carboxylic acid with an aliphatic chain, which is either saturated or unsaturated. Examples of fatty acid include, with limitation to, caprylic acid (C8), capric acid (C10), lauric acid (C12), myristic acid (C14), myristoleic acid (C14:1), palmitic acid (C16), stearic acid (C18), palmitoleic acid (C16:1), oleic acid (C18:1), linoleic acid (C18:2) and α-linolenic acid (18:3).

The "fatty acid derivative" refers to the vinyl-, methyl-, ethyl-, isoamyl-, benzyl- or isopropyl- ester of the following fatty acids: caprylic acid (C8), capric acid (C10), lauric acid (C12), myristic acid (C14), myristoleic acid (C14:1), palmitic acid (C16), stearic acid (C18), palmitoleic acid (C16:1), oleic acid (C18:1), linoleic acid (C18:2) and α-linolenic acid (18:3).

In a preferred embodiment of the method of the invention the fatty acid chain comprising between 9 to 16 carbon atoms, preferably between 10 to 15 carbon atoms.

In a preferred embodiment of the method of the invention the fatty acid chain comprising 11, 12, 13 or 14 carbon atoms, preferably the fatty acid chain is a 12 carbon atoms chain, more preferably is a lauric acid.

In an even more preferred embodiment of the method of the invention, vinyl laurate (derivative of the lauric acid) is used in step ii).

In a preferred embodiment of the method of the invention. the acylated derivative of phloretin 4'-O-α-D-glucopyranoside is phloretin 4'-O-(6-O-lauroyl)-α-O-glucopyranoside.

Particularly, the acylation of the 4'-O-α-D-glucopyranoside is carried out in *tert*-butyl alcohol and in presence of vinyl laurate. The concentration of vinyl laurate can be between 30 and 360 mM (preferred value 320 mM), which corresponds to a molar ratio between 2:1 to 22:1 with respect to the concentration of 4'-O-α-D-glucopyranoside.

In a preferred embodiment of the method of the invention, the reaction of step (i), the α-glucosylation, is carried out at a temperature range between 35°C and 60°C.

In other preferred embodiment of the method of the invention, the reaction of step (i) is carried out at a temperature between 40ºC to 55 ºC.

In other more preferred embodiment of the method of the invention, the temperature of the reaction of step (i) is 41ºC, 42ºC, 43ºC, 44ºC, 45ºC, 46ºC, 47ºC, 48ºC, 49ºC, 50ºC, 51ºC, 52ºC, 53ºC or 54ºC, preferably is 48ºC.

In other preferred embodiment of the method of the invention, the reaction of step (ii), the acylation, is carried out at a temperature range between 50°C and 70°C, preferably between 55ºC and 65ºC.

In other more preferred embodiment of the method of the invention the temperature of the reaction of step (i) is 56ºC, 57ºC, 58ºC, 59ºC, 60ºC, 61ºC,62ºC, 63ºC or 64ºC, preferably is 60ºC.

In other preferred embodiment of the method of the invention the reaction time of step (i) is between 10 and 70 hours.

In other more preferred embodiment of the method of the invention the reaction time of step (i) is between 5 to 60 hours, preferably is between 10 to 40 hours, more preferably is between 10 to 30 hours, even more preferably 10 to 24 hours.

In other even more preferred embodiment of the method of the invention the reaction time of step (i) is 11, 12, 13, 14, 15, 16, 17, 18, 19, 20, 21, 22 or 23 hours, preferably 12 hours.

In other preferred embodiment of the method of the invention the step (i) is reach out with orbital shaking between 600 to 1200rpm, preferably between 750 to 1050 rpm.

### DESCRIPTION OF THE DRAWINGS

**Figure 1****.** Reaction pathway proposed to obtain a new compound with better skin absorption score, where (A) is the enzymatic synthesis of phloretin 4'-O-α-D-monoglucoside using the sucrose phosphorylase mutant TtSPP_R134A from *Thermoanaerobacterium thermosaccharolyticum,* and (B) is the subsequent acylation strategy with a commercial lipase from *Thermomyces lanoginosus,* selective to react with the group 6-OH of the glucose, obtaining the expected product regio-selectively.

**Figure 2****.** Phloretin monoglucoside acylation chromatograms (HPLC) after 0 h, 1 h and 6 h. The reaction was carried out employing a comercial lipase (Lipozyme TL IM) from *Thermomyces lanoginosus.*

**Figure 3****.** Kinetic study of phloretin monoglucoside acylation. Reaction conditions: Phloretin 4'-O-α-glucopyranoside (7 mg, 16 mM) and vinyl laurate (84 µL, 320 mM) in tert-butanol (1 mL), with Lipozyme TL IM (7 mg) at 60 °C and a vigorous shaking.

**Figure 4****.** Molecular structure of the obtained compound: Phloretin 4'-O-(6-O-lauroyl)-α-D-glucopyranoside.

**Figure 5****.** *In vitro* percutaneous absorption of several phloretin derivatives within the skin layers. "W" is the surface excess, "SC" is the stratum corneum, "E" is the epidermis, "D" is the dermis, "FR" is the fluid receptor and "Perc. Abs." is the percutaneous absorption. The results were expressed as µg/cm2 (mean values ± standard deviations, n = 3, *p < 0.05). The percutaneous absorption of the acylated derivative was compared with the absorption of the phloretin and the phloretin monoglucoside previously reported (Gonzalez-Alfonso, J. L., et al, 2021, Advanced Synthesis & Catalysis, 363 (n/a), 3079-3089).

### EXAMPLES

### 1. MATERIALS AND METHODS

### Enzyme and reagents

For the production of recombinant sucrose phosphorylase mutant (TtSPP_R134A) from *Thermoanaerobacterium thermosaccharolyticum,* the encoding gene was ligated into the expression plasmid pCXshP34. Then, the plasmid was transformed into E. *coli* BL21 cells and said cells were grown at 37 ºC (with continuous shaking at 200 rpm) in 5 mL of LB medium with 5 µL of ampicillin (100 mg/mL) during 8 h. Next, the preculture was added to 500 mL of LB medium contained in a 2 L Erlenmeyer flask with 500 µL of ampicillin. The culture was grown overnight at 37 ºC with continuous shaking at 200 rpm. Subsequently, the cells were centrifuged at 10,000 rpm and 4ºC during 15 min, and the cell pellet was frozen at -20ºC. To extract the enzyme from the cells, the frozen cell pellet was slowly thawed on ice, and the cells were suspended in lysis buffer, containing lysozyme (1 mg/mL) and 1% of 10 mM phenylmethanesulfonyl fluoride (PMSF) in 50 mM MOPS buffer at pH 6.5. The suspended cells were subsequently disrupted by sonication (Branson 250 Sonifier, level 3, 50% duty cycle) 3 times during 3 min each cycle. The cell debris was removed by centrifugation for 30 min at 4,000 rpm and 4ºC, and the supernatant (crude cell extract) containing the enzyme was collected. The enzyme was semipurified using heat purification, by incubating the crude cell extract in a water bath at 60ºC during 30 min. After this step, the mixture was centrifuged at 4,000 x g during 30 min and the supernatant was recovered and stored at 4ºC.

Lipozyme TL IM (250 IUN/g) is a commercial lipase from *Thermomyces lanuginosus* (EC 3.1.1.3) immobilized on granulated silica and was provided by Novozymes S.A.

Vinyl laurate, ABTS [2,2'-azino-bis (3-ethylbenzothiazoline-6-sulphonic acid)], DPPH (2,2-diphenyl-1-picrylhydrazyl) and (R)-Trolox (6-hydroxy-2,5,7,8-tetramethylchroman-2-carboxylic acid) were acquired in Sigma Aldrich. All other reagents and solvents were of the highest purity grade available.

### General procedure for Phloretin Glucosylation

For the α-glucosylation reaction, 10 mg of phloretin in 200 µL of acetone were mixed with 684 mg of sucrose in 1400 µL of 50 mM MOPS buffer (pH 6.5) and 200 µL of sucrose phosphorylase TtSP_R134A (10U). The total volume was 2 mL. The reaction was maintained at 48 °C during 24 h with orbital shaking (800 rpm) and followed by thin-layer chromatography (TLC) and high-performance liquid chromatography (HPLC). The TLC analysis was carried out with 60 F₂₅₄ silica gel plates (Merck) with a mixture of ethyl acetate, methanol and H₂O (30:5:4) as mobile phase. The plate was revealed under UV light and using 10% of H₂SO₄ solution. HPLC analysis was performed using a quaternary pump (model 600, Waters, Milford, MA, USA) coupled to an autosampler (model ProStar 420, Varian Inc., Palo Alto, CA, USA). The column was a Zorbax Eclipse Plus C18 (4.6 x 100 mm, 3.5 µm, Agilent) at 40ºC and the detector was a photodiode array (ProStar, Varian). Peaks were detected at 297 nm and analyzed with the software Varian Star LC workstation 6.41. The mobile phase was composed of water and acetonitrile, both solvents with 0.2% (v/v) of formic acid, and the flow rate was 0.8 mL/min. The gradient started with 15% (v/v) of acetonitrile. The acetonitrile percentage was increased to 40% (v/v) within 5 min, keeping this percentage constant during 5 min. After that, the mobile phase returned to the initial conditions and the column was equilibrated for 5 min.

### Purification of Glucosylated Derivatives of Phloretin

The reaction was scaled up to purify the glucosylated products. The reaction mixture contained 100 mg of phloretin in 2 mL of acetone, 6.84 g of sucrose in 17 mL of 50 mM MOPS buffer (pH 6.5) and sucrose phosphorylase TtSPP_R134A (65 U). The mixture was kept at 48 °C during 56 h with orbital shaking (1000 rpm), and the glucosylation progress was followed by TLC and HPLC as described. When the concentration of the monoglucoside derivative reached a value of approximately 4.3 mg/mL, the reaction was stopped with methanol (20 mL) and the solvent evaporated with a rotary evaporator (model R- 210, Büchi; Flawil, Switzerland). The purification was carried out using a silica gel 60 (particle size 0.06-0.2 mm, Scharlau) chromatographic column after extraction steps of phenolic compounds with 2-butanone (5 x 20 mL) to remove the residual sugars. The solutions with suspended silica were evaporated with the rotary evaporator and the product was adsorbed in the silica. The mobile phase consisted of a mixture of ethyl acetate, methanol and water at a ratio of 30:5:4 (v/v/v). The different fractions were collected in test tubes and the purification was followed by TLC with the method previously described. Finally, the solvents were evaporated to obtain the pure products.

### General procedure for enzymatic acylation of phloretin 4'-O-α-D-glucopyranoside

Phloretin 4'-O-α-D-glucopyranoside (7 mg, 16 µmοl), vinyl laurate (84 µL, 320 µmοl), and Lipozyme TL IM (7 mg) were mixed in tert-butyl alcohol (1 mL). The reaction was carried out at 60ºC under orbital shaking between 600 to 1200 rpm. Aliquots of 50 µL were taken at different times and diluted with 450 µL of methanol. Samples were analysed by Thin Layer Chromatography (TLC) and High-performance Liquid Chromatography (HPLC). TLC analysis was carried out with silica gel plates 60 F254 (Merck) with a mixture of ethyl acetate, methanol and water 60:5:4 (v/v/v) as mobile phase. The spots were first visualized by UV light and then employing 10% (v/v) H₂SO₄ solution and heating the plate. HPLC analysis was performed using a quaternary pump (model 600, Waters, Milford, MA, USA) coupled to an autosampler (model ProStar 420, Varian Inc., Palo Alto, CA, USA). The column was a Zorbax Eclipse Plus C18 (4.6 x 100 mm, 3.5 µm, Agilent) at 40ºC. The detector was a photodiode array (ProStar, Varian), and peaks were detected at 297 nm and analyzed with the software Varian Star LC workstation 6.41. The mobile phase was acetonitrile and water in gradient, both solvents acidified with 0.2 % (v/v) of formic acid. The gradient was formed by increasing acetonitrile from 15 % to 95 % in 5 min, followed by an isocratic step of 10 min at 95 %. After that, the column was equilibrated during 5 min at initial conditions before the next injection.

### Product purification

The reaction was scaled-up 5-fold under the described conditions (total volume of 5 mL). After 24 h, the solvent was evaporated with a rotavapor (R-210, Buchi), and the pellet was washed with toluene and water, to separate the residual lauric acid and phloretin monoglucoside. After that, the solid residue was dissolved in methanol. The main product was purified by silica-gel chromatography, employing a mixture ethyl acetate, methanol and water 60:5:4 (v/v/v) as mobile phase. The different fractions were collected and the purification was followed by TLC as described above. Finally, the solvents were evaporated yielding the acylated phloretin α-D-glucoside.

### Mass Spectrometry (MS)

The molecular weight of the main product was identified by mass spectrometry with electrospray ionization (ESI) coupled to a hybrid QTOF analyzer (model MAXIS II, Bruker) in positive reflector mode. Methanol with 0.1 % (v/v) of formic acid was employed as the ionizing phase.

### Nuclear Magnetic Resonance (NMR)

The structure of the product was determined using a combination of 1D and 2D (COSY, DEPT-HSQC, NOESY) standard NMR techniques. The spectra of the sample, dissolved in DMSO-d6 (ca. 7 mM), were recorded on a Bruker IVDr 600 spectrometer equipped with a BBI probe with gradients in the Z axis, at a temperature of 300 K. Chemical shifts were expressed in parts per million (ppm). Residual DMSO-d5 signal was used as an internal reference (2.5 ppm). All the employed pulse sequences were provided by Bruker. For the DEPT-HSQC experiment, values of 8 ppm and 1K points, for the 1H dimension, and 165 ppm and 256 points for the 13C dimension, were used. For the homonuclear COSY and NOESY experiments, 8 ppm windows were used with a 1K × 256 point matrix. For the NOESY the mixing time was 500 ms.

### Phloretin 4'-O-α-D-Glucopyranoside

The compound (1) was obtained as a pinkish powder (58.3 mg,33%); 1H NMR (CD₃OD) δ: 2.86 (2H, m, CH2-β), 3.30 (2H, m, CH2-α), 3.45 (1H, dd, J=8.94, 9.96 Hz, H4"), 3.54 (1H, ddd, J=2.58, 4.38, 9.96 Hz, H5"), 3.57 (1H, dd, J=3.63, 9.75 Hz, H2"), 3.70 (1H, dd, J=4.35, 12.15 Hz, H6"a), 3.74 (1H, dd, J=2.58, 12.06 Hz, H6"b), 3.81 (1H, dd, J=9.09, 9.57 Hz, H3"), 5.53 (1H, d, J=3.60 Hz, H1"), 6.17 (2H, s, H3'/H5'), 6.69 (2H, d, J=8.50 Hz, H2/H6), 7.04 (2H, d, J=8.50 Hz, H3/H5); 13C NMR (CD₃OD) δ: 31.2 (CH2 β), 47.4 (CH2 α), 62.0 (C6"), 71.0 (C4"), 73.0 (C2"), 74.6 (C5"), 74.8 (C3"), 96.4 (C3'/C5'), 98.4 (C1"), 107.0 (C1'), 115.9 (C3/C5), 130.3 (C2/C6), 133.8 (C1), 156.6 (C4), 164.5 (C4'), 165.7 (C2'/C6'), 207.3 (CO); HPLC-UV (297 nm): t*_{R}* 9.36 min (90%); ESI-MS (m/z) 459.1264 [M+Na]⁺.

### Phloretin 4'-O-α-D-(glucopyranoside 6"-laurate)

After silica gel chromatographic column purification, the product was collected as a yellow oil (34.2 mg, 67 %) ¹H NMR (CD₃OD) δ: δ: 0.89 (3H, m, H12a), 1.17-1.35 (12H, m, H4a-9a), 1.27 (2H, m, H10a), 1.3 (2H, m, H11a), 1.46 (2H, m, H3a), 2.2 (2H, t, H2a), 2.86 (2H, m, H7), 3.3 (1H, dd J=9.3, 10.0 Hz, H4"), 3.3 (2H, m, H8), 3.6 (1H, dd, J=3.7, 9.7 Hz, H2"), 3.7 (1H, ddd, J=2.1, 7.8, 10.0 Hz, H5"), 3.8 (1H, dd, J=9.3, 9.7, H3"), 4.1 (1H, m, H6"), 4.4 (1H, m, H6"), 5.5 (1H, d, J=3.7 Hz, H1"), 6.2 (2H, s, H3'/H5'), 6.7 (2H, d, H3/H5), 7.0 (2H, d, H2/H6). ¹³C NMR (CD₃OD) δ: 14.3 (C12a), 25.7 (C3a), 26.8 (C11a), 29-31 (C4a-9a), 31.3 (C7), 33.0 (C10a), 35.0 (C2a), 47.3 (C8), 64.5 (C6"), 71.9 (C4"), 72.4 (C5"), 72.9 (C2"), 74.8 (C3"), 96.8 (C3/C5), 97.8 (C1"), 106.9 (C1'), 116.1 (C3/C5), 130.1 (C2/C6), 133.7 (C1), 156.3 (C4), 164.4 (C4'), 165.3 (C2'/C6'), 175.2 (C1a), 206.9 (C9) HPLC-UV (297 nm): t*_{R}* 10.7 min (89%). ESI-MS (m/z) 641.2925 [M+Na]⁺.

### Aqueous solubility

The compound was incubated at 25ºC in water (saturated conditions) during 3 days under orbital stirring (1000 rpm). Then, the sample was centrifuged. A volume of 200 µL of the supernatant solution was analysed by triplicate, in a 96-well plate, using a phloretin calibration curve in methanol (0-200 µg/mL). The absorbance was measured by UV spectrophotometry (Tecan Infinite M200) at 297 nm.

### Antioxidant activity

The capacity of the synthesized ester to reduce 2,2-diphenyl-1-picrylhydrazyl (DPPH) and the radical cation 2,2-azino-bis(3-ethylbenzothiazoline-6-sulfonic acid) diammonium salt (ABTS^{·+}) was assayed. (R)-Trolox was employed as antioxidant reference. Standard solutions with a concentration between 0 and 1000 µM for DPPH assay, and between 0 and 500 µM for ABTS assay, were prepared.

### Percutaneous activity methodology

The study was carried out in vitro with pig biopsies placed on Franz static diffusion cells (3 mL, 1.86 cm² of exposed area, diameter: 30 mm, Lara-Spiral, Courtenon, France) in order to determine the distribution of active compound (API) at the different skin layers, after an exposure time of 24 hours. The pig skin, provided by the Department of Cardiology of the Hospital Clinic (Barcelona, Spain), is considered morphologically and functionally equivalent to human skin for percutaneous absorption studies. Ultra-pure water was employed as receptor fluid, and cells were placed in a thermostated water bath (32 °C) containing a magnetic stirring device to obtain the skin surface temperature at 32 ± 1ºC. The integrity of the skin samples was evaluated by measuring the transepidermal water loss (TEWL) with Tewameter TM300 (Courage & Khazaka, Cologne, Germany) considering correct TEWL values under 15 g m⁻² h⁻¹. Solutions with active compound [phloretin, phloretin 4'-O-α-D-glucopyranoside (monoglucoside) and phloretin 4'-O-α-D-(glucopyranoside 6"-laurate) (acylated monoglucoside)] were applied (10.75 µL/cm²) on dermatomized porcine skin biopsies.

Experiments were done by triplicate in three diffusion cells, and an extra diffusion cell without any active compound was used as control. After 24 h, the skin surface was washed to recover the excess of compounds (W). Then, the receptor fluid (FR) was recovered, and stripping procedures were performed on the surface horny layers of the stratum corneum (SC) with adhesive tape (D-squame, Cuderm Corporation, Dallas, USA). Eight strips were employed in order to remove most of the amount of substance contained in the stratum corneum. The viable epidermis (E) was separated from the dermis (D) after heat treatment. The different samples to be analyzed (W, SC, E, D and FR) were extracted and/or diluted in methanol (Merck, Darmstadt, Germany). The phloretin, its corresponding monoglucoside and acylated monoglucoside concentrations were analyzed by HPLC, as previously described. The substance is considered absorbed when is present in epidermis (except for the stratum corneum), dermis and receptor fluid. The amounts founded in the stratum corneum are not considered to be percutaneously absorbed (Perc. Abs). The results are shown as mean values (expressed as µg/cm2) ± standard deviations. The Kruskal-Wallis test was applied for group comparisons, with the software STATGRAPHICS^{®}, assuming significant differences when *p < 0.05.*

### 2. RESULTS

### Phloretin 4'-O-α-D-(glucopyranoside) acylation

The reaction was carried out by mixing phloretin 4'-O-α-glucoryranoside (7 mg, 16 mM) and vinyl laurate (84 µL, 320 mM) in *tert*-butanol (1 mL) and the subsequent addition of Lipozyme TL IM (7 mg). The reaction progress was followed by Thin Layer Chromatography (TLC). A new spot appeared after a short time with higher Rf than the substrate which could correspond to an acylated derivative. The enzymatic biotransformation was also followed by high-performance liquid chromatography (HPLC) by taking aliquots at different times. The resulting chromatograms are shown in Figure 2.

The progress of formation of the main product was followed by HPLC (Figure 3). The reaction reached the total conversion of phloretin glucoside into the desired product (approx. 10 mg/mL, >99% conversion yield) after 6 hours. The efficiency of the enzymatic reaction is really high because of the presence of the glucose moiety in the molecule. Furthermore, the use of different long chain acyl donors could provide a way to modulate the physicochemical properties of the derivatives, as well as their biological properties in terms of *in vivo* absorption, bioavailability and solubility.

### Chemical characterization of the acylated derivative

The acylated derivative was purified by silica-gel chromatography and characterized by Mass Spectrometry (ESI-MS) and Nuclear Magnetic Resonance (NMR). The inventors observed the presence of major pick in ESI-MS with m/z 641.2925 which fitted with [M + Na]+ ion of the expected product. The acylation position of the acylated derivative was confirmed by NMR. 1D-selective spectra (NOESY/TOCSY) were performed. The molecular structure of the obtained novel compound, Phloretin 4'-O-(6-O-lauroyl)-α-D-glucopyranoside, is represented in Figure 4.

### Solubility

The aqueous solubility of the acyl phloretin monoglucoside derivative was analysed at 25ºC during 72 h and compared with the results previously obtained with phloretin monoglucoside and the aglycone in Table 1. The acylation of the monoglucoside decreased the aqueous solubility.

**Table 1. Water solubility at 25ºC of phloretin, phloretin monoglucoside and the novel acylated derivative. (*) Data available in Gonzalez-Alfonso, J. L., et al, 2021, Advanced Synthesis & Catalysis, 363 (n/a), 3079-3089.**

| **Solubility (mg/L)** | |
|---|---|
| Phloretin | 23.2 ± 0.3* |
| Phloretin 4'-O-α-D-(glucopyranoside) | 1644 ± 101 * |
| Phloretin 4'-O-α-D-(glucopyranoside 6"-laurate) | 6.0 ± 0.2 |

### Antioxidant properties

The antioxidant activity of the acyl-monoglucoside was evaluated measuring the Trolox Equivalent Antioxidant Capacity (TEAC) value on ABTS and DPPH. The results are summarized in Table 2.

**Table 2. Antioxidant activity on ABTS and DPPH of the obtained acylated product. Data is expressed as EC50 ± SD and TEAC value ± SD (n = 3). The TEAC value was calculated from EC50 of the acyl derivative and EC50 of the (R)-Trolox as reference compound. (*) Data available in Gonzalez-Alfonso, J. L., et al, 2021, Advanced Synthesis & Catalysis, 363 (n/a), 3079-3089.**

| | **ABTS assay** | | **DPPH assay** | |
|---|---|---|---|---|
| | *EC50 (µM)* | *TEAC* | *EC50 (µM)* | *TEAC* |
| Phloretin* | 11.7 ± 0.6 | 0.45 ± 0.04 | 217 ± 14 | 6.2 ± 0.4 |
| Phloretin 4'-O-α-D-glucopyranoside* | 16.9 ± 0.5 | 0.64 ± 0.06 | 285 ± 20 | 8.1 ± 0.6 |
| Phloretin 4'-O-α-D-(glucopyranoside 6"-laurate) | 14.7 ± 0.7 | 0.47 ± 0.04 | 439 ± 13 | 11.4 + 0.8 |

These results can be compared with the phloretin and the phloretin 4'-O-α-D-(glucopyranoside) values previously. The high capacity of the acyl derivative to reduce ABTS^{·+} is in concordance with the same assay for phloretin and the monoglucosylated derivative.

### Skin absorption

The inventors explored the enzymatic acylation as a strategy to increase the skin absorption of polyphenolic compounds. The capacity of phloretin to protect against UV radiation, its antimicrobial activity and its antioxidant power make the phloretin an interesting compound in cosmetics. Previously, the inventors reported the skin absorption of phloretin, a monoglucoside and a diglucoside (Gonzalez-Alfonso, J. L., et al, 2021, Advanced Synthesis & Catalysis, 363 (n/a), 3079-3089*).* Phloretin and its monoglucoside displayed the same absorption pattern, without significant differences. On the other hand, the diglucoside derivative absorption was diminished, providing an interesting antioxidant and soluble compound to protect the external skin layers. However, if the objective is to increase the percutaneous absorption allowing the compound to penetrate through the skin barriers, the simple glycosylation is not a good strategy.

In contrast, the acylation of the glucosides increases the skin absorption. Figure 5 represents the results of skin absorption of the synthesized acylated derivative of phloretin using pig skin biopsies. The graphic also shows the skin absorption of the aglycone (phloretin) and the phloretin glucoside at 4'-OH, reported in the previous work (Gonzalez-Alfonso, J. L., et al, 2021, Advanced Synthesis & Catalysis, 363 (n/a), 3079-3089*).*

The acyl-glucoside of phloretin exhibited a greater (approx. 3-fold increase) percutaneous absorption than the other compounds. This result demonstrates that enzymatic glucosylation followed by acylation is a good strategy to enhance the skin absorption of phenolic compounds.

### 3. CONCLUSIONS

The present invention refers a useful two-step strategy for the synthesis of acylated derivatives of polyphenols. The first step is the α-glucosylation of the aglycone by a sucrose phosphorylase mutant, which gives rise to an excellent yield and regioselectivity with many polyphenols, including phloretin. The glucose moiety provides an acylation point for regioselective lipases, such as that from *Thermomyces lanuginosus.* Using this strategy, a very high yield of acylated α-glucoside of phloretin has been obtained. Further, the phloretin 4'-O-(6-O-lauroyl)-α-D-glucopyranoside is more efficient to penetrate through a skin model, which opens the door to develop tailor-made products for cosmetic applications.

## Claims

1. A compound of formula (I): 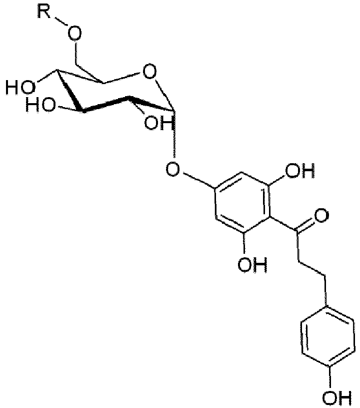 wherein R is a -(CO)-C₇-C₁₇ linear alkyl or -(CO)-C₇-C₁₇ linear alkenyl.

2. The compound according to claim 1 of formula (II):

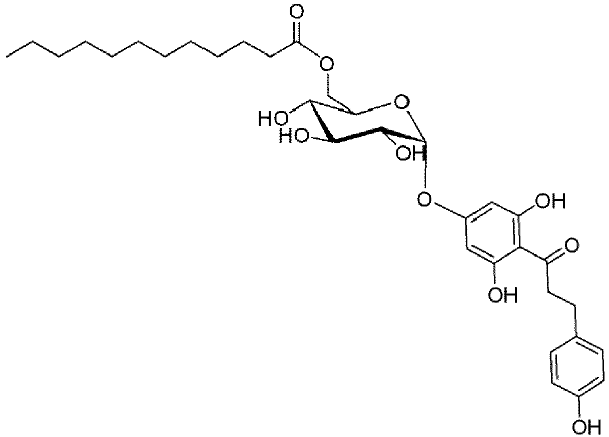

3. A composition comprising the compound of formula (I) or (II) according to claim 1 or 2.

4. The composition according to claim 3 wherein the composition is a cosmetic composition.

5. The composition according to claim 4 wherein said composition further comprises a cosmetically acceptable excipient or vehicle.

6. The composition according to any one of claims 3 to 5 wherein the composition is formulated for administration in liquid or solid form.

7. Cosmetic use of the compound according to claim 1 or 2 or the composition according to any one of claims 3 to 6.

8. Method for producing an acylated derivative of phloretin 4'-O-α-D-glucopyranoside, as described in claim 1 or 2 comprising:
(i) contacting the phloretin (2',4',6'-trihydroxy-3-(4-hydroxyphenyl)-propiophenone) with a mutant of the sucrose 6'-phosphate phosphorylase from *Thermoanaerobacterium thermosaccharolyticum,* in aqueous media and in presence of sucrose, wherein the said mutant of the sucrose 6'-phosphate phosphorylase comprises the amino acid sequence of SEQ ID NO: 2, and
(ii) contacting the phloretin 4'-O-α-D-glucopyranoside obtained in step (i) with an immobilized lipase from *Thermomyces lanuginosus,* in organic solvent and in presence of a fatty acid with a chain comprising between 8 to 18 carbon atoms or a derivative thereof, obtaining the acylated derivative of phloretin 4'-O-α-D-glucopyranoside.

9. The method according to claim 8 wherein the fatty acid chain is a lauric acid, preferably is a derivative of the lauric acid.

10. The method according to claim 8 or 9 wherein the acylated derivative of phloretin 4'-O-α-D-glucopyranoside is phloretin 4'-O-(6-O-lauroyl)-α-D-glucopyranoside.

11. The method according to any one of the claims 8 to 10 wherein the reaction of step (i) is carried out at a temperature range between 35°C and 60°C and/or the reaction of step (ii) is carried out at a temperature range between 50°C and 70°C, preferably the reaction of step (i) is carried out at a temperature of 48ºC and/or the reaction of step (ii) is carried out at a temperature of 60ºC.

12. The method according to any one of claims 8 to 11 wherein the reaction time of step (i) is between 10 and 70 hours, preferably between 10 to 24 hours, and with orbital shaking between 600 to 1200 rpm, preferably between 750 to 1050 rpm.

13. Use of of the compound according to claim 1 or 2 or the composition according to any one of claims 3 to 6 for manufacturing cosmetic products.
